# EUROPÄISCHE PATENTSCHRIFT

(11) **EP 0 661 065 B1**
(45) Veröffentlichungstag und Bekanntmachung des Hinweises auf die Patenterteilung: **07.07.1999**
(21) Anmeldenummer: 94120253.3
(22) Anmeldetag: 21.12.1994
(51) Int. Cl.: A61M 5/172

(54) **Infusionssystem mit Regeleinrichtung**
Infusion system comprising a control device
Système de perfusion avec dispositif de réglage

(30) Priorität: 29.12.1993 DE 4344872
(43) Veröffentlichungstag der Anmeldung: 05.07.1995
(73) Patentinhaber: B. BRAUN MELSUNGEN AG, 34212 Melsungen (DE)
(72) Erfinder: Röher, Otfried, Prof. Dr. Ing., D-01139 Dresden (DE); Korth, Steffen, D-99086 Erfurt (DE); Belke, Roberto, D-22119 Hamburg (DE)
(74) Vertreter: Selting, Günther, Dipl.-Ing.

(56) Entgegenhaltungen:
- EP-A- 0 408 483

## Beschreibung

Die Erfindung betrifft ein Infusionssystem mit einer Regeleinrichtung, die die Medikamentendosierung automatisch an die multifaktoriellen Einflüsse des sich zeitlich verändernden Patientenzustandes anpaßt.

Die Infusion von Medikamenten stellt in vielen Fällen eine unentbehrliche Therapiemaßnahme zur Aufrechterhaltung der Vitalfunktionen des menschlichen Organismus dar. Insbesondere während der extrakorporalen Blutwäsche von chronisch Nierenkranken, nach größeren Operationen und bei Verletzungen mit hohem Blutverlust sind für die häufig auftretenden Komplikationen multifaktorielle Einflüsse mit hohen Nichtlinearitäten charakteristisch. Die Wirkmechanismen der einzelnen Einflußfaktoren sind zwar qualitativ bekannt, ihr komplexes Zusammenwirken jedoch weitgehend unerforscht. Die kausalen Zusammenhänge der Komplikationen sind somit durch einen hohen Anteil unscharfen Wissens gekennzeichnet.

Zahlreiche Infusionssysteme sind wegen ihrer deterministischen Regelcharakteristiken und der zugehörigen Algorithmen auf das scharfe (deterministische) Wissen über die Regelmechanismen des menschlichen Organismus beschränkt.

In mehreren bekannten Fällen liegt den verwendeten Reglern ein Black-Box-Modell zugrunde, das unter stationären oder quasistationären Bedingungen gültig ist. Derartige Regler reagieren deshalb nicht oder nur ungenügend auf die spontanen Sensitivitätsänderungen des körpereigenen Regulationssystems. Weitere Nachteile ergeben sich bei Prozessen mit längeren Totzeiten, wie sie in der Regel im menschlichen Organismus bei Infusion von Medikamenten auftreten.

In weiteren bekannten Infusionssystemen werden Reglerstrukturen mit adaptiven Eigenschaften verwendet. Die Adaption der Infusionsrate an den sich verändernden Patientenzustand erfolgt dabei mittels fest vorgegebener Algorithmen, die teilweise auch eine Vorabschätzung für den Zeitraum bis zum Wirkungseintritt des Medikamentes ermöglichen. Die Algorithmen wurden anhand einzelner Wirkkomponenten der multifaktoriellen Regelmechanismen erstellt. Diese Regler erreichen deshalb nur solange brauchbare Ergebnisse, wie die zugrundegelegten Kausalzusammenhänge tatsächlich den aktuellen Wirkungsverlauf bei Medikamenteninfusion bestimmen.

Wie die medizinische Praxis zeigt, sind jedoch die im menschlichen Organismus auftretenden Komplikationen häufig auf qualitative Veränderungen im komplexen Zusammenwirken der multifaktoriellen Komponenten zurückzuführen. Diese Phänomene umfassen somit mehrere semantische Domänen, die nur als unscharfes (probabilistisches) Wissen in Form von probabilistischen Relationen und linguistischen Variablen darstellbar sind.

Für derartige Einsatzfälle sind deshalb Infusionssysteme erforderlich, die mehrere qualitativ unterschiedliche Einflußkomponenten und ihre semantischen Domänen als unscharfes Wissen erfassen und auswerten können sowie unter Berücksichtigung ihrer semantischen Domänenbewertung in die automatische Regelung der Medikamentendosierung einbeziehen. Dies betrifft insbesondere auch solche medizinischen Einsatzgebiete wie die extrakorporale Blutwäsche, bei der sich zusätzliche Einflußgrößen unmittelbar aus den therapeutischen Zielvorgaben der Behandlung ergeben. Hierbei müssen die Wechselwirkungen zwischen der Medikamentendosierung und den Zielgrößen der Behandlung wie Ultrafiltrationsrate, Gesamtflüssigkeitsentzug, Natriumbilanz und andere als wichtige Regelparameter laufend berücksichtigt werden.

Die genannten Regler sind aufgrund ihrer deterministischen Struktur prinzipiell nicht imstande, diese Anforderungen zu erfüllen.

In der Chirurgie sind Blutdruckregler für die intra- und postoperative Überwachung bekannt, die auf der Grundlage der Fuzzy-Logik unscharfes Wissen verarbeiten können. (H. Ying et al. Expert-system-based fuzzy control of arterial pressure by drug infusion in: Medical Progress through Technology, Kluner Academic Publishers, Dordrecht, Netherlands, 1988, Seiten 203 bis 215).

Aus EP-A-408 483 ist ein Medikamentenzuführungssystem mit einer Regeleinrichtung bekannt, das ausschließlich für die Blutdruckregelung mittels Infusion vasoaktiver Medikamente bestimmt ist. Dieses Infusionssystem enthält deshalb zur Ermittlung der Infusionsrate nur Meßwerterfassungs- und Verarbeitungsmodule für den Kausalzusammenhang zwischen dem vasoaktiven Medikament, der hierdurch verursachten Veränderung des Spannungszustandes der Blutgefäße und der resultierenden Blutdruckänderung. Die Eingangsgrößen sind deshalb auf die von einem Blutdruckmonitor ermittelten Parameter des Blutdruckverlaufes beschränkt.

Diese fuzzygeregelten Infusionssysteme erfüllen aus folgenden Gründen ebenfalls nicht die genannten Anforderungen: Die medizinische Zielstellung der Blutdruckregelung besteht ausschließlich darin, den Blutdruck während der prä-, der intra- und der postoperativen Phase mittels Infusion eines vasoaktiven Medikaments auf dem Sollwert konstant zu halten. Als Entscheidungsmerkmale des Fuzzy-Reglers werden nur Parameter des Blutdruckverlaufs wie der systolische und/oder mittlere Blutdruck, die Abweichung vom Blutdruck-Sollwert (Fehler), die zeitliche Änderung (Rate) und die Blutdruckfrequenz (Puls) verwendet. Die Fuzzy-Regelung beschränkt sich damit auf nur eine semantische Domäne. Diese fuzzygeregelten Infusionssysteme berücksichtigen nicht mehrere semantische Domänen für unterschiedliche physiologische Wirkungsmechanismen.

Aufgabe der Erfindung ist deshalb die Schaffung eines Infusionssystems mit einer Regeleinrichtung, die mehrere Einflußgrößen mit unterschiedlichen physiologischen Wirkungsmechanismen, d.h. mehrere semantische Domänen, als unscharfes Wissen aktiv in die automatische Medikamentendosierung einbezieht und dabei die unterschiedliche semantische Bedeutung der einzelnen Domänen berücksichtigt.

Die Aufgabe ist bei einem Infusionssystem nach dem Oberbegriff des Anspruchs 1 erfindungsgemäß durch die im Kennzeichen des Anspruchs 1 angegebenen Merkmale gelöst. Vorteilhafte Weiterbildungen der Erfindung sind Gegenstand der Unteransprüche. Erfindungsgemäß sind bei dem Infusionssystem mit einer Regeleinrichtung zur Steuerung mindestens einer Infusionsvorrichtung mehrere Fuzzy-Regler in einer hierarchischen Systemstruktur vorgesehen.

Die ermittelten Meßgrößen und/oder linguistischen Variablen werden von einer zentralen Meßwertverarbeitungseinheit erfaßt und von dieser werden weitere Größen abgeleitet, die für die Auswertung von essentieller Bedeutung sind.

Sämtliche linguistischen Variablen, Meßgrößen und abgeleiteten Größen werden in einer ersten Hierarchie-Ebene mittels eines Semantikanalysators hinsichtlich ihrer inhaltlichen Bedeutung für die verschiedenen semantischen Domänen untersucht. Der Semantikanalysator enthält einen ersten Fuzzy-Regler, der alle Eingangsgrößen mit ihren zugehörigen Fuzzy-Sets verknüpft (Fuzzyfizierung) und mittels integrierter Fuzzy-Logik in jeder semantischen Domäne die Gesamtzugehörigkeiten für alle in der zugehörigen Regelbasis definierten Zustände ermittelt.

Durch eine ebenfalls integrierte Einheit zur semantischen Domänenbewertung werden hieraus in mehreren Bewertungsstufen Schlußfolgerungen für die Arbeitsweise einer zweiten Hierarchie-Ebene gezogen, in der jede semantische Domäne durch einen eigenen weiteren Fuzzy-Regler repräsentiert ist.

Die semantische Gesamtbewertung jeder einzelnen Domäne wird vom Semantikanalysator der ersten Hierarchie-Ebene als scharfe Steuergröße (Defuzzyfizierung) an den zugehörigen weiteren Fuzzy-Regler der zweiten Hierarchie-Ebene übergeben. Weiterhin erhält jeder weitere Fuzzy-Regler selektiv alle Eingangsgrößen, die von der Meßwertverarbeitungseinheit für seine Domäne ermittelt wurden.

Jeder Fuzzy-Regler verfügt außerdem über eine domänenspezifische Regelbasis und Module zur Fuzzyfizierung, logischen Verknüpfung, Fuzzy-Inferenz und Defuzzyfizierung.

Durch die hierarchische Struktur und Arbeitsweise des Infusionssystems wird bei der automatischen Medikamentendosierung eine approximative Widerspiegelung der ärztlichen Entscheidungsprozesse erreicht. Durch die kontinuierliche Semantikanalyse in der ersten Hierarchie-Ebene und die selektive Steuerung der Fuzzy-Regler in der zweiten Hierarchie-Ebene wird insbesondere gewährleistet, daß die qualitativ unterschiedlichen Einflußkomponenten sowie die im Laufe der Behandlung eintretenden Veränderungen in ihrem komplexen Zusammenwirken entsprechend ihrer semantischen Bedeutung erkannt und in die automatische Medikamentendosierung einbezogen werden.

Im folgenden wird ein Ausführungsbeispiel unter Bezugnahme auf die Zeichnungen näher erläutert.

Es zeigen:
- Fig. 1: ein Ausführungsbeispiel eines Infusionssystems für die extrakorporale Blutwäsche von chronisch Nierenkranken,
- Fig. 2a bis 2c: Schaubilder der drei semantischen Domänen Hypotonie, Plasmavolumen und Ultrafiltration einschließlich ihrer Subdomänen.
- Fig. 3: die Zugehörigkeitsfunktionen (Fuzzy-Sets) von drei Variablen, die als Entscheidungsmerkmale im ersten Fuzzy-Regler verwendet werden,
- Fig. 4: die Ermittlung des Schwerpunktes und Momentes der aktivierten Zustände, und
- Fig. 5: die Arbeitsweise der Domänenbewertungs-Einheit.

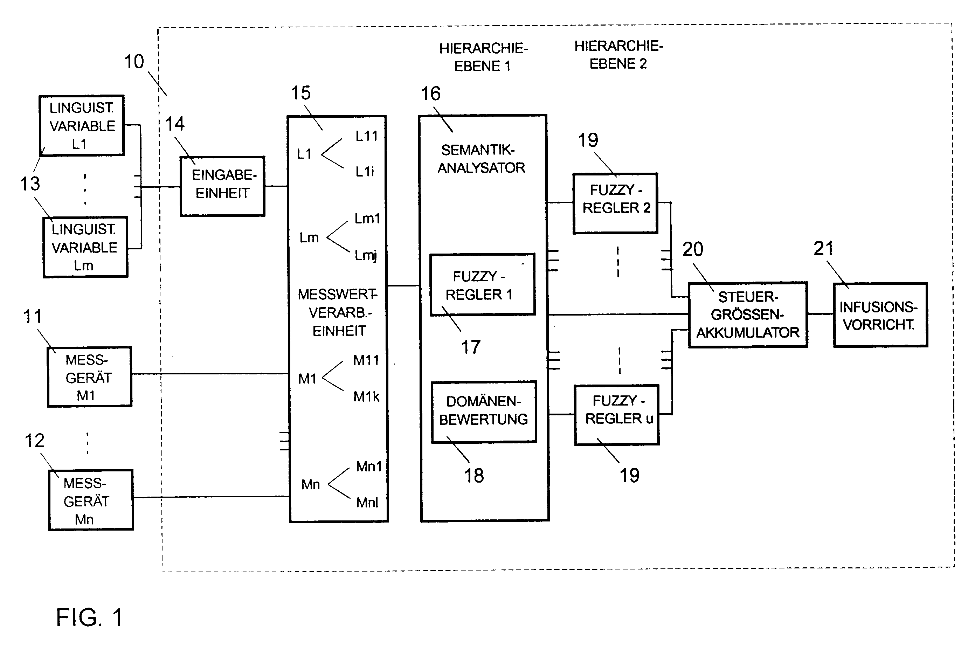

Das Infusionssystem nach Fig. 1 verfügt über eine hierarchisch strukturierte Regeleinrichtung 10, an die n verschiedene Meßgeräte 11,12 angeschlossen sind. Da das Infusionssystem zur Blutdruckstabilisierung während der extrakorporalen Blutwäsche dient, wird als Meßgerät 11 ein Blutdruckmeßgerät verwendet, das als Meßgröße M₁ den systolischen und/oder mittleren arteriellen Blutdruck des Patienten mißt.

Mit den weiteren Meßgeräten 12 werden andere Meßgrößen M₂...Mₙ ermittelt, die für die Blutdruckstabilisierung während der extrakorporalen Blutwäsche relevant sind. Hierfür kommen zunächst körpereigene Parameter in Betracht, die über physiologische Prozesse auf den Blutdruck einwirken, wie z.B. Plasmavolumen, Hämatokrit, Puls, Herzminutenvolumen und Elektrolytkonzentrationen.

Weiterhin können Meßgrößen einbezogen werden, die während der extrakorporalen Blutwäsche über den transmembranen Stoffaustausch auf den Blutdruck einwirken, wie z.B. Ultrafiltratmenge und -rate, Dialysatzusammensetzung und -temperatur, Natriumbilanz und Medikamentenverbrauch.

Sprachliche Informationen 13, wie z.B. subjektive Beschwerden des Patienten, ärztliche Kommentare und zusätzliche Therapieanweisungen, werden als linguistische Variable L₁...Lₘ über eine Eingabeeinheit 14, vorzugsweise eine Tastatur, in die Regeleinrichtung 10 eingegeben.

Von der Meßwertverarbeitungseinheit 15 werden alle Eingangsgrößen L₁...Lₘ und M₁...Mₙ erfaßt und daraus weitere, für die Beurteilung des Blutdruckverlaufes wesentliche Größen L₁₁...Lₘⱼ und M₁₁...Mₙ₁ als Eingangsgrößen für einen Semantikanalysator 16 abgeleitet, wie z.B. Differenzen zwischen Meßwerten, Änderungsgeschwindigkeiten, Kurz- und Langzeittrends.

Sämtliche von der Meßwertverarbeitungseinheit erfaßten und abgeleiteten Größen werden dem Semantikanalysator 16 zugeführt, der ihre inhaltliche Bedeutung für die verschiedenen semantischen Domänen ermittelt und die erste Hierarchie-Ebene der Regeleinrichtung darstellt. Da für die semantischen Zusammenhänge zwischen den Eingangsgrößen und den semantischen Domänen kein scharfes (deterministisches) medizinisches Wissen existiert, wird für die Semantikanalyse ein erster Fuzzy-Regler 17 verwendet.

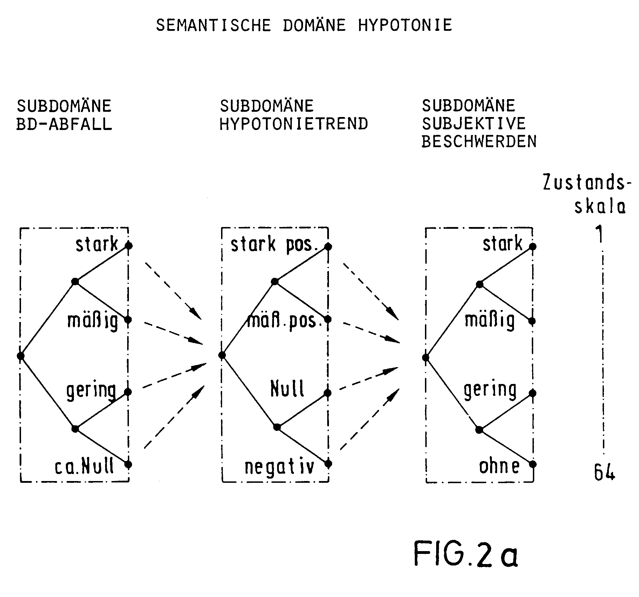

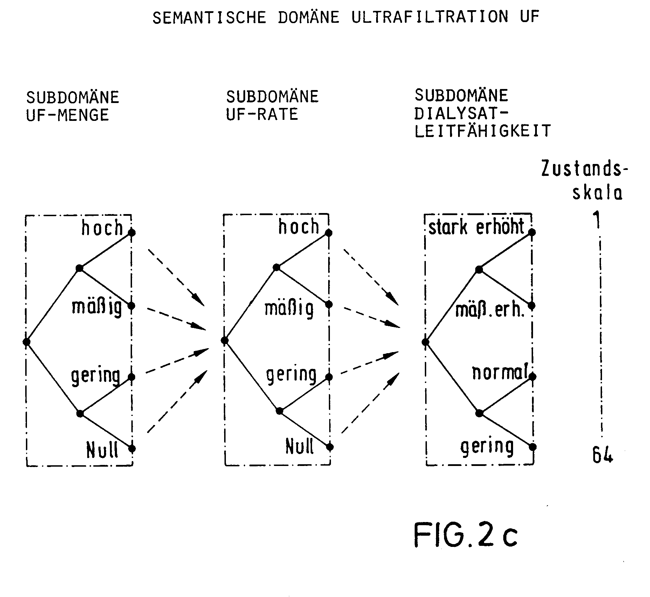

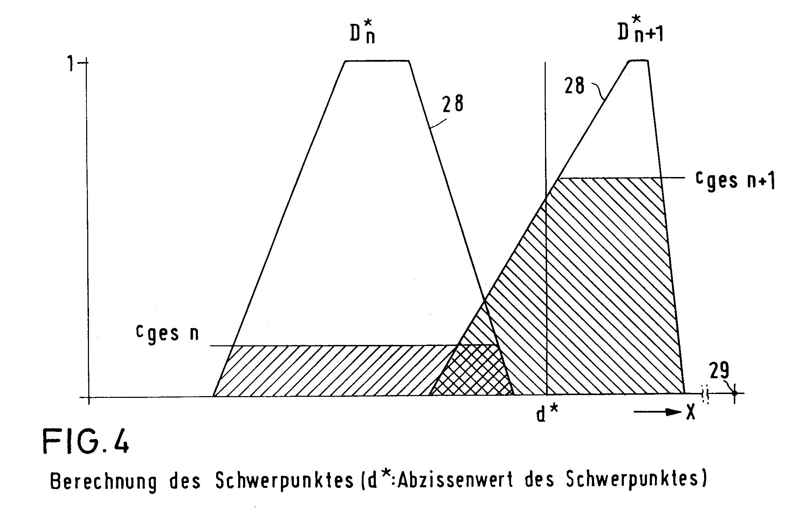

Fig. 2a zeigt eine schematische Darstellung der im ersten Fuzzy-Regler 17 verarbeiteten semantischen Domäne Hypotonie. Die Domäne ist in die Subdomämen Blutdruckabfall (BD-Abfall), Hypotonietrend und subjektive Beschwerden aufgeteilt und enthält somit 64 (4³) verschiedene Zustände. Die auf dieser Grundlage entwickelte Regelbasis des Fuzzy-Reglers besteht dementsprechend aus einer Zustandsskala mit 64 Regeln. Jedem Zustand ist entsprechend seiner Bedeutung für den Blutdruckverlauf eine durch die zugehörige Regel festgelegte Medikamentendosis D*ₙ in Form eines Fuzzy-Sets zugeordnet, wie es Fig. 4 am Beispiel zweier Regeln D*ₙ und D*ₙ₊₁ zeigt. Die x-Achse stellt den gesamten Dosisbereich dar, über den sich die 64 Regeln entsprechend ihrer Dosis verteilen.

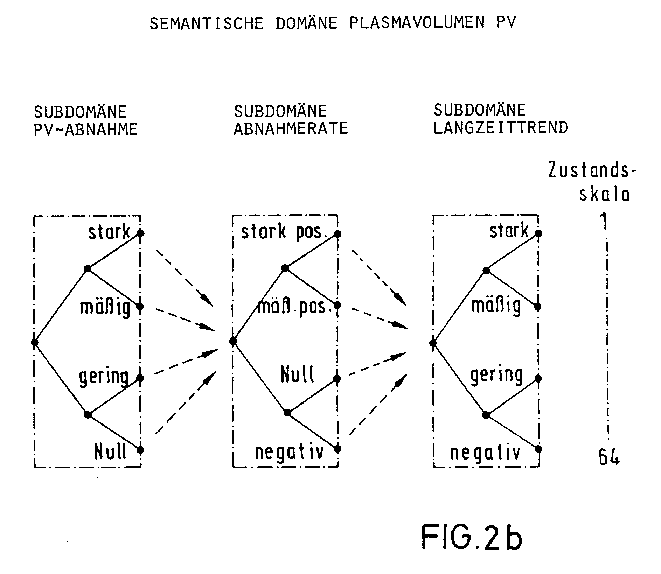

Die Vielfalt der blutdruckrelevanten Einflußkomponenten veranschaulichen die semantischen Domänen Plasmavolumen in Fig. 2b und Ultrafiltration in Fig. 2c, wobei in beiden Domänen im Gegensatz zu Fig. 2a der Blutdruck selbst nicht als Entscheidungskriterium auftritt.

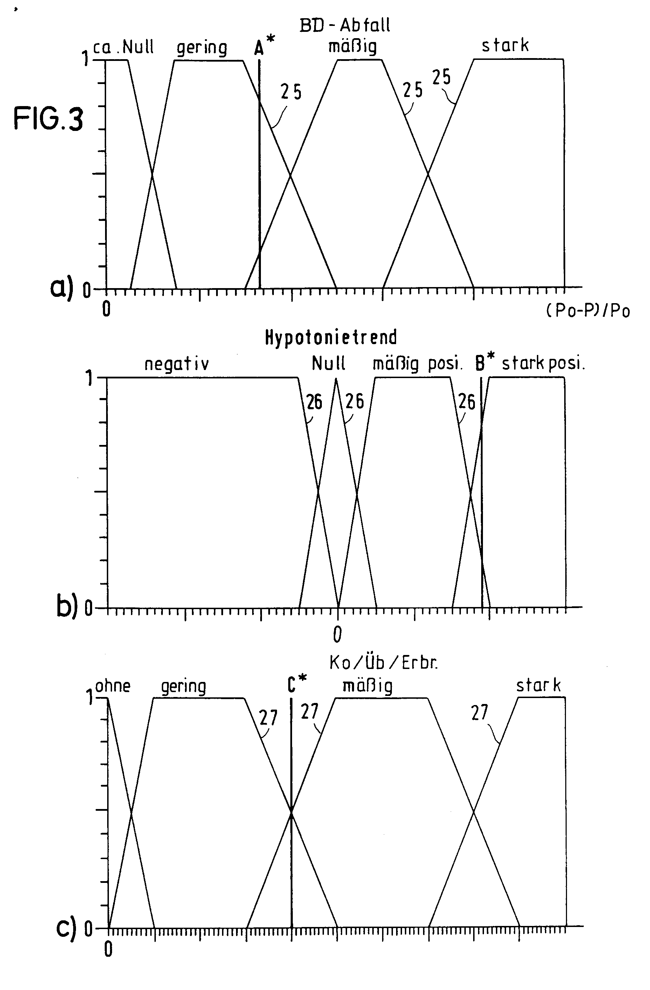

Fig. 3 zeigt am Beispiel der Zugehörigkeitsfunktionen von drei verschiedenen Entscheidungskriterien, wie die Fuzzy-Sets für unscharfes Wissen im Fuzzy-Regler 17 gespeichert sind. In Fig. 3a sind die Zugehörigkeitsfunktionen 25 für das Entscheidungskriterium Blutdruckabfall (BD-Abfall) dargestellt. Entlang der Abszisse ist der relative Blutdruckabfall (P_{O}-P)/P_{O} aufgetragen, wobei bei P_{O} der vorgewählte Soll-Blutdruck und P der gegenwärtig gemessene Blutdruck sind. Entlang der Ordinate sind die Zugehörigkeitswerte c in einer Skala von "0" bis "1" aufgetragen. Dem Entscheidungskriterium BD-Abfall sind vier linguistische Variablen als Merkmalsintervalle in Form von Fuzzy-Sets zugeordnet, nämlich die Intervalle "ca. Null", "gering", "mäßig" und "stark". Jedes dieser Merkmalsintervalle ist trapezförmig, wobei die Trapeze symmetrisch oder asymmetrisch sein können und sich gegenseitig überlappen.

Bei einem bestimmten aktuellen Blutdruckabfall, der in Fig. 3a als A* angegeben ist, ergeben sich aus den Schnittpunkten der Linie A* mit den Zugehörigkeitsfunktionen die Zugehörigkeitswerte für die jeweiligen linguistischen Variablen.

In Fig. 3b sind die Zugehörigkeitsfunktionen 26 für das Kriterium Hypotonietrend dargestellt. Die vier linguistischen Variablen sind "negativ", "Null", "mäßig positiv" und "stark positiv". Auch hier erfolgt die Fuzzyfizierung durch trapez- bzw. dreieckförmige Zugehörigkeitsfunktionen im Wertebereich 0...1. Der aktuelle Wert sei mit B* bezeichnet. Im dargestellten Beispiel ergeben sich aus den Schnittpunkten von B* Zugehörigkeitswerte >0 mit den Fuzzy-Sets "mäßig positiv" und "stark positiv".

Durch das dritte Entscheidungskriterium Subjektive Beschwerden in Fig. 3c werden Angaben über Kopfschmerz, Übelkeit und Erbrechen erfaßt, die mit einer Bewertung ihres Stärkegrades in die Tastatur 14 eingegeben werden. Der aktuelle Wert C* für dieses Entscheidungskriterium markiert im gezeigten Beispiel gleichgroße Zugehörigkeitswerte von 0,5 für die Merkmalsintervalle "gering" und "mäßig". Die Zugehörigkeitsfunktionen von Fig. 3c sind mit 27 bezeichnet.

Dem komplexen Zusammenwirken mehrerer Einflußgrößen aus unterschiedlichen semantischen Domänen wird im Semantikanalysator 16 durch einen mehrstufigen Inferenzprozeß zur semantischen Bewertung der Domänen Rechnung getragen. Hierzu werden in der ersten Bewertungsstufe vom Fuzzy-Regler 17 die nach Fig. 3 ermittelten Zugehörigkeiten zu den einzelnen Merkmalsintervallen für die 64 möglichen Zustände mittels eines Mengenoperators, beispielsweise des Minimum-Operators, zu Gesamtzugehörigkeiten c_{ges n} (n = 1 ...64) aggregiert. Alle Zustände, für die sich sowohl aus den aktuellen Fakten A* wie auch B* und C* Zugehörigkeitswerte >0 ergeben, werden dabei als aktivierte Zustände mit einer Gesamtzugehörigkeit >0 ausgewiesen.

Die erste semantische Bewertung der einzelnen Domänen wird hieraus durch Berechnung des Schwerpunktes und des Momentes der Gesamtzugehörigkeiten aller aktivierten Zustände der Domäne ermittelt. Das Moment einer semantischen Domäne ergibt sich aus der Lage des Schwerpunktes aller Gesamtzugehörigkeiten dieser Domäne.

Fig. 4 veranschaulicht die Berechnung des Schwerpunktes einer Domäne und seines Momentes am Beispiel von zwei aktivierten Zuständen D*ₙ und D*ₙ₊₁, wobei n hier eine Zahl von 1 bis 64 auf der Zustandsskala ist und die Nummer des Zustandes in der betreffenden Domäne angibt. Jeder Zustand 28 ist durch ein trapezförmiges Fuzzy-Set definiert, dessen Lage auf der x-Achse seinen Dosisbereich markiert. Die beiden schraffierten Flächen entsprechen den Gesamtzugehörigkeiten c_{ges n} und c_{ges n+1}, die aus den Schnittpunkten der aktuellen Fakten A*,B* und C* mit den Fuzzy-Sets der betreffenden Domäne ermittelt werden. Aus der Größe der schraffierten Flächen und ihrer Lage auf der x-Achse werden der Schwerpunkt und das Moment beider Zustände berechnet. d* ist der Abszissenwert des Schwerpunktes der beiden schraffierten Flächen, und das Moment ist das Produkt des Abstandes von d* vom Endwert 29 der Zustandsskala und dem Flächeninhalt der schraffierten Flächen.

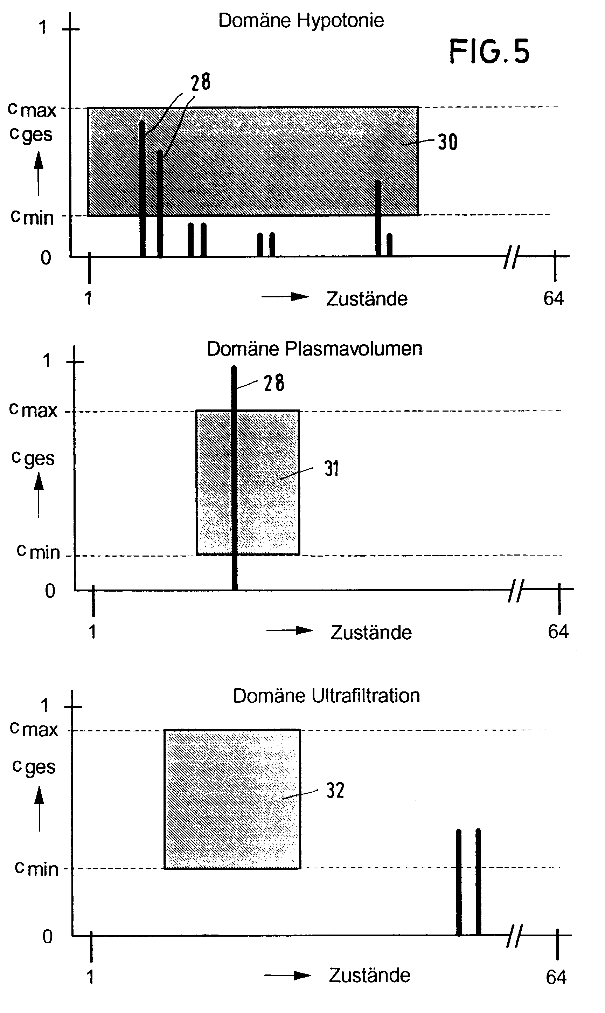

Für die weiteren semantischen Bewertungsstufen verfügt der Semantikanalysator 16 über eine Domänenbewertungs-Einheit 18, deren Arbeitsweise Fig. 5 anhand der in Fig. 2a-c dargestellten semantischen Domänen Hypotonie, Plasmavolumen und Ultrafiltration veranschaulicht. In der Domänenbewertungs-Einheit 18 ist das ärztliche Wissen über den medizinischen Stellenwert der verschiedenen Zustände und die Schlußfolgerungen abgespeichert, die unter Berücksichtigung der ermittelten Momente der einzelnen Domänen zu ziehen sind. In der zweiten semantischen Bewertungsstufe erfolgt eine Gewichtung jeder einzelnen Domäne mit einem festgelegten Faktor. Im Ergebnis der zweiten semantischen Bewertungsstufe wird durch die Domänenbewertungs-Einheit 18 festgelegt, in welchem Umfang die Fuzzy-Regler der zweiten Hierarchie-Ebene an der Berechnung der von der angeschlossenen Infusionsvorrichtung 21 zu applizierenden Medikamentenmengen bzw. Infusionsraten beteiligt werden.

Nachdem der Fuzzy-Regler 17 in der ersten semantischen Bewertungsstufe die Momente aller semantischen Domänen ermittelt hat, werden gemäß Fig. 5 in der zweiten semantischen Bewertungsstufe die Gesamtzugehörigkeiten c_{ges n} der aktivierten Zustände bezüglich ihrer Lokalisation in bezug auf semantisch determinierte Felder 30,31,32 analysiert, die für die ärztlichen Entscheidungen zur Dosierung vorrangige Bedeutung haben. Die Linien 28 in Fig. 5 entsprechen den Gesamtzugehörigkeiten c_{ges}, wie sie in Fig. 4 durch die schraffierten Flächen der Zustände 28 markiert sind. Sobald mindestens eine Gesamtzugehörigkeit ein solches Feld 30,31,32 berührt, erhält das Moment der betreffenden Domäne einen Wichtungsfaktor >1. Die Zahlenwerte dieser Wichtungsfaktoren können sich domänenspezifisch unterscheiden. Beträgt beispielsweise in Fig. 5 das Moment der Domäne Hypotonie 0,8 und der vorbestimmte Wichtungsfaktor 2, so ergibt sich ein gewichtetes Moment von 1,6. Liegen andererseits alle aktivierten Zustände einer Domäne außerhalb des schraffierten Feldes, wie in der Domäne Ultrafiltration gezeigt, wird der Wichtungsfaktor gleich 1 gesetzt, so daß das Moment und das gewichtete Moment dieser Domäne identisch sind.

In der dritten semantischen Bewertungsstufe analysiert die Domänenbewertungs-Einheit 18, ob Gesamtzugehörigkeiten c_{ges n} existieren , die einen vorgegebenen Maximalwert cₘₐₓ überschreiten und gleichzeitig ein schraffiertes Feld schneiden, wie in Fig. 5 in der Domäne Plasmavolumen skizziert. Diese dritte semantische Bewertungsstufe läßt sowohl Wichtungsfaktoren analog zur zweiten Bewertungsstufe wie auch kontroverse Wichtungen zu, wenn ein stark aktivierter Zustand, wie in der Domäne Plasmavolumen skizziert, aus ärztlicher Sicht eine vorwiegend oder ausschließlich plasmavolumengesteuerte Infusion erfordert. Eine ausschließlich plasmavolumen-gesteuerte Infusion kann zum Beispiel in der dritten Bewertungsstufe erreicht werden, indem für die Domäne Plasmavolumen der Wichtungsfaktor 1 verwendet wird und für die Domänen Hypotonie und Ultrafiltration durch einen Wichtungsfaktor 0 deren bisherige Wichtungen aufgehoben werden. Die Tabelle erläutert den beschriebenen Sachverhalt anhand einfacher Zahlenbeispiele:

| Semantische Domäne | Erste Bewertung Moment | Zweite Bewertung vorbest. | | Dritte Bewertung zusätzl. | |
|---|---|---|---|---|---|
| | | Faktor | gewicht. Moment | Faktor | Semant. Gesamt-Bewert. |
| Hypotonie | 0,8 | 2,0 | 1,6 | 0 | 0 |
| Plasmavolumen | 0,6 | 4,0 | 2,4 | 1,0 | 2,4 |
| Ultrafiltration | 0,1 | 1,0 | 0,1 | 0 | 0 |

Obiges Beispiel beschreibt einen Extremfall. In der Regel ergeben sich für die semantische Gesamtbewertung in allen Domänen Werte >0.

Die mit 19 bezeichneten Fuzzy-Regler 2 bis u in der zweiten Hierarchie-Ebene multiplizieren ihre domänenspezifischen Inferenzergebnisse automatisch mit dem in der letzten (rechten) Tabellenspalte ausgewiesenen Wert für die semantische Gesamtbewertung ihrer Domäne. Der Steuergrößenakkumulator 20 faßt die Ergebnisse der einzelnen Domänen zusammen und bildet daraus die Gesamt-Steuergröße für die angeschlossene Infusionsvorrichtung 21. Hierzu wird die Summe aller semantischen Bewertungen gleich 100% gesetzt und die Inferenzergebnisse der einzelnen Fuzzy-Regler 2...u mit dem entsprechenden Prozentanteil ihrer Domäne multipliziert. Durch diese Normierung auf 100% wird gewährleistet, daß die vorgegebene Maximaldosis auch bei hohen Dosisanteilen der einzelnen Domänen nie überschritten werden kann. Die Summe der normierten Prozentanteile aller Domänen gibt unmittelbar die von der Infusionsvorrichtung 21 zu applizierende Medikamentenmenge oder Infusionsrate an.

## Patentansprüche

1. Infusionssystem mit einer Regeleinrichtung (10) zur Steuerung mindestens einer Infusionsvorrichtung (21), wobei die Regeleinrichtung (10) unscharfes Wissen mittels linguistischer Variablen und Methoden der Fuzzy-Logik verarbeitet,
**dadurch gekennzeichnet,**
daß die Regeleinrichtung (10) eine Meßwertverarbeitungseinrichtung (15), einen Semantikanalysator (16) mit einem ersten Fuzzy-Regler (17) sowie mit einer Bewertungseinheit (18), weitere Fuzzy-Regler (19) und einen Steuergrößenakkumulator (20) aufweist,
daß die Meßwertverarbeitungseinheit (15) Eingangsgrößen (L₁₁...Lₘⱼ; M₁₁... Mₙ₁ für den Semantikanalysator (16) aus Meßwerten (M_{1...}Mₙ) und/oder linguistischen Variablen (L₁...Lₘ) erzeugt,
daß der erste Fuzzy-Regler (17) die Eingangsgrößen (L₁₁...Lₘⱼ;M₁₁...Mₙ₁) zu semantischen Domänen zusammenfaßt und in jeweils einer Zustandsskala zuordnet, wobei der Fuzzy-Regler (17) jede Domäne anhand gespeicherter Fuzzy-Sets (25,26,27) und der Zustandsskala analysiert, indem er für jeden einzelnen Zustand der Zustandsskala den Zugehörigkeitswert (c_{ges}) ermittelt,
daß die Bewertungseinheit (18) die Zugehörigkeitswerte (C_{ges}) domänenspezifisch wichtet, wobei die Bewertungseinheit (18) für jede Domäne eine semantische Bewertung erzeugt,
daß für jede Domäne einer der weiteren Fuzzy-Regler (19) vorgesehen ist, der aus den Zugehörigkeitswerten (c_{ges}) der Domäne und aus der semantischen Bewertung eine domänenspezifische Steuergröße erzeugt,
und daß der Steuergrößenakkumulator (20) die domänenspezifischen Steuergrößen mehrerer Domänen zu einer Gesamt-Steuergröße für die Infusionsvorrichtung (21) zusammenfaßt.

2. Infusionssystem nach Anspruch 1, dadurch gekennzeichnet, daß der Semantikanalysator (16) aus den Zugehörigkeitswerten (c_{ges}) einer Domäne durch Schwerpunktbildung ein Moment bildet und zur Erzeugung der semantischen Bewertung wichtet.

3. Infusionssystem nach Anspruch 2, dadurch gekennzeichnet, daß die gewichteten Momente aller Domänen die semantischen Bewertungen bilden.

4. Infusionssystem nach Anspruch 3, dadurch gekennzeichnet, daß die Bewertung der Zugehörigkeitswerte (c_{ges}) mit Faktoren erfolgt, deren Größe davon abhängt, ob in einem semantisch determinierten Feld (31,32,33) des Zugehörigkeitsdiagramms der Domäne Zugehörigkeitswerte vorhanden sind.

5. Infusionssystem nach Anspruch 4, dadurch gekennzeichnet, daß ein Zugehörigkeitswert einen zusätzlich erhöhten Faktor erhält, wenn er einen vorgegebenen Grenzwert übersteigt und im Bereich des semantisch determinierten Feldes liegt.

## Claims

1. An infusion system comprising a control means (10) for controlling at least one infusion device (21), the control means (10) processing undetermined knowledge by means of linguistic variables and fuzzy-logic methods,
**characterized in**
that the control means (10) comprises a measurement value processing means (15), a semantic analyzer (16) with a first fuzzy controller (17) and with an evaluation means (18), further fuzzy controllers (19) and a control-value accumulator (20),
that the measurement value processing means (15) generates input values (L₁₁...Lₘⱼ; M₁₁...Mₙ₁) for the semantic analyzer (16) from measurement values (M₁...Mₙ) and/or linguistic variables (L₁...Lₘ),
that the first fuzzy controller (17) combines the input values (L₁₁...Lₘⱼ; M₁₁...Mₙ₁) into semantic domains and assigns them to respectively one condition scale, with the fuzzy controller (17) analyzing each domain by means of stored fuzzy sets (25,26,27) and the condition scale by determining the grade-of-membership (c_{ges}) for each individual state of the condition scale,
that the evaluation means (18) performs a domain-specific weighting of the grades-of-membership (c_{ges}), with the evaluation means (18) generating a semantic evaluation for each domain,
that, for each domain, one of the further fuzzy controllers (19) is provided for generating a domain-specific control value from the grades-of-membership (c_{ges}) of the domain and from the semantic evaluation,
and that the control-value accumulator (20) combines the domain-specific control values of a plurality of domains into a total control value for the infusion device (21).

2. The infusion system according to claim 1, characterized in that the semantic analyzer (16), by generating a center of gravity, forms a moment from the grades-of-membership (c_{ges}) of a domain and weightens said moment for generating the semantic evaluation.

3. The infusion system according to claim 2, characterized in that the weighted moments of all domains form the semantic evaluation.

4. The infusion system according to claim 3, characterized in that the evaluation of the grades-of-membership (c_{ges}) is performed by factors, the amounts of said factors depending on whether or not grades-of-membership are present in a semantically determined field (31,32,33) of the membership diagram of the domain.

5. The infusion system according to claim 4, characterized in that a grade-of-membership, when exceeding a predetermined limit value and being within the range of said semantically determined field, obtains an additionally increased factor.

## Revendications

1. Système de perfusion comprenant un dispositif de régulation (10) destiné à commander au moins un dispositif de perfusion (21), le dispositif de régulation (10) traitant des connaissances imprécises au moyen de variables linguistiques et de méthodes de la logique floue, caractérisé
en ce que le dispositif de régulation (10) comporte un dispositif de traitement des valeurs de mesure (15), un analyseur de sémantique (16) avec un premier régulateur à logique floue (17) ainsi qu'avec une unité d'évaluation (18), d'autres régulateurs à logique floue (19) et un accumulateur de grandeurs de commande (20),
en ce que l'unité de traitement des valeurs de mesure (15) élabore des grandeurs d'entrée (L₁₁...Lₘⱼ ; M₁₁...Mₙ₁) pour l'analyseur de sémantique (16), à partir de valeurs de mesure (M₁...Mₙ) et/ou de variables linguistiques (L₁...Lₘ),
en ce que le premier régulateur à logique floue (17) regroupe les grandeurs d'entrée (L₁₁...Lₘⱼ ; M₁₁...Mₙ₁) en domaines sémantiques et les associe respectivement dans une échelle d'états, le régulateur à logique floue (17) analysant chaque domaine sur la base d'ensembles d'éléments de logique floue (25, 26, 27) mémorisés et de l'échelle d'états, en déterminant pour chaque état individuel de l'échelle d'états, la valeur d'appartenance (c_{ges}), en ce que l'unité d'évaluation (18) pondère les valeurs d'appartenance (c_{ges}) de manière spécifique par domaine, l'unité d'évaluation (18) élaborant une évaluation sémantique pour chaque domaine,
en ce que pour chaque domaine, est prévu l'un des autres régulateurs à logique floue (19), qui élabore, à partir des valeurs d'appartenance (c_{ges}) du domaine et de l'évaluation sémantique, une grandeur de commande spécifique au domaine,
et en ce que l'accumulateur de grandeurs de commande (20) regroupe les grandeurs de commande, spécifiques au domaine, de plusieurs domaines, en une grandeur de commande globale pour le dispositif de perfusion (21).

2. Système de perfusion selon la revendication 1, caractérisé en ce que l'analyseur de sémantique (16), à partir des valeurs d'appartenance (c_{ges}) d'un domaine, forme, par établissement de barycentre, un moment, et le pondère pour l'élaboration de l'évaluation sémantique.

3. Système de perfusion selon la revendication 2, caractérisé en ce que les moments pondérés de tous les domaines forment les évaluations sémantiques.

4. Système de perfusion selon la revendication 3, caractérisé en ce que l'évaluation des valeurs d'appartenance (c_{ges}) est effectuée avec des facteurs dont la grandeur dépend de la présence ou non de valeurs d'appartenance dans un champ déterminé sémantiquement (31, 32, 33) du diagramme d'appartenance du domaine.

5. Système de perfusion selon la revendication 4, caractérisé en ce qu'un facteur augmenté d'un supplément est affecté à une valeur d'appartenance, lorsqu'elle dépasse une valeur limite prescrite et se situe dans la zone du champ déterminé sémantiquement.
